(19) Europäisches Patentamt
European Patent Office
Office européen des brevets

(11) EP 1 861 091 B1

(12) **EUROPEAN PATENT SPECIFICATION**

(45) Date of publication and mention of the grant of the patent:
**11.12.2013 Bulletin 2013/50**

(21) Application number: **06704954.4**

(22) Date of filing: **03.03.2006**

(51) Int Cl.:
***A61K 31/355*** (2006.01) ***A61K 31/375*** (2006.01)
***A61K 31/07*** (2006.01) ***A61P 3/10*** (2006.01)
***A61P 9/10*** (2006.01) ***A61P 3/06*** (2006.01)
***A61P 9/00*** (2006.01) ***A61P 13/12*** (2006.01)
***A61P 1/16*** (2006.01) ***A61P 35/00*** (2006.01)
***A61K 31/122*** (2006.01) ***A61K 31/661*** (2006.01)
***A61K 31/683*** (2006.01) ***A61K 31/665*** (2006.01)
***A61K 45/06*** (2006.01)

(86) International application number:
**PCT/AU2006/000281**

(87) International publication number:
**WO 2006/092025 (08.09.2006 Gazette 2006/36)**

(54) **COMPOUNDS HAVING LIPID LOWERING PROPERTIES**
VERBINDUNGEN MIT LIPIDSENKENDEN EIGENSCHAFTEN
COMPOSÉS AYANT DES PROPRIÉTÉS D'ABAISSEMENT DES LIPIDES

(84) Designated Contracting States:
**AT BE BG CH CY CZ DE DK EE ES FI FR GB GR HU IE IS IT LI LT LU LV MC NL PL PT RO SE SI SK TR**

(30) Priority: **03.03.2005 AU 2005901013**

(43) Date of publication of application:
**05.12.2007 Bulletin 2007/49**

(73) Proprietor: **Vital Health Sciences Pty Ltd.**
**Melbourne, VIC 3000 (AU)**

(72) Inventors:
- **WEST, Simon, Michael**
  **Williamstown, VIC 3016 (AU)**
- **OGRU, Esra**
  **Wheelers Hill VIC 3150 (AU)**
- **LIBINAKI, Roksan**
  **Chadstone, VIC 3148 (AU)**

(74) Representative: **Vossius & Partner**
**Siebertstrasse 4**
**81675 München (DE)**

(56) References cited:
**EP-A1- 0 617 963** **EP-A1- 0 617 963**
**EP-A1- 0 674 904** **EP-A1- 0 681 840**
**EP-A1- 1 053 749** **EP-A1- 1 973 547**
**EP-A2- 0 430 045** **EP-A2- 0 643 969**
**WO-A1-03/026673** **WO-A1-03/068209**
**WO-A1-2004/064831**

- **MUNTEANU A ET AL: "Modulation of cell proliferation and gene expression by alpha-tocopheryl phosphates: relevance to atherosclerosis and inflammation" BIOCHEMICAL AND BIOPHYSICAL RESEARCH COMMUNICATIONS, ACADEMIC PRESS INC. ORLANDO, FL, US, vol. 318, no. 1, 21 May 2004 (2004-05-21), pages 311-316, XP004504106 ISSN: 0006-291X**
- **MUNTEANU A. ET AL.: 'Modulation of cell proliferation and gene expression by alpha-tocopheryl phosphates: relevance to atherosclerosis and inflammation' BIOCHEMICAL AND BIOPHYSICAL RESEARCH COMMUNICATIONS vol. 318, no. 1, 21 May 2004, pages 311 - 316, XP004504106**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 861 091 B1

## Description

### Field of the invention

The invention relates to one or more phosphate derivatives of one or more electron transfer agents for use in a method for lowering increased circulating blood levels of one or more of the following lipids: LDL cholesterol, triglycerides and overall cholesterol.

### Background of the invention

Cardiovascular disease (CVD) which includes heart disease and stroke is the number one cause of death in Western societies. This is believed to be due to a number of factors including, excessive proliferation of vascular smooth muscle cells (SMC), elevated total cholesterol and low density lipoprotein (LDL) cholesterol. Although changes in lifestyle, including diet and exercise, are recommended first lines of intervention, drug therapy is not only often used, it is also often warranted.

While often thought of as the same thing, heart and cardiovascular disease are different, involving different parts of the body. Heart disease refers only to diseases of the heart and the blood vessel system within the heart. Cardiovascular disease refers to diseases of the heart and diseases of the blood vessel system (arteries, capillaries, veins) within a person's entire body, such as the brain, legs, and lungs. "Cardio" refers to the heart and "vascular" refers to the blood vessel system.

The heart is a strong, muscular pump slightly larger than a fist. It pumps blood continuously through the circulatory system, the network of elastic tubes that allows blood to flow throughout the body. The circulatory system includes two major organs, the heart and lungs, and blood vessels (arteries, capillaries, and veins). Arteries and capillaries carry oxygen- and nutrient-rich blood from the heart and lungs to all parts of the body. Veins carry oxygen- and nutrient-depleted blood back to the heart and lungs. Heart and blood vessel problems do not happen quickly. Over time, the arteries that bring blood to the heart and brain can become blocked from a build up of cells, fat, and cholesterol (plaque). Reduced blood flow to the heart from blockages in the arteries causes heart attacks. Lack of blood flow to the brain from a blood clot, or bleeding in the brain from a broken blood vessel, causes a stroke.

There are many forms of heart and cardiovascular disease, and what follows is a list of the most common of these diseases.

- Coronary heart disease (or coronary artery disease).
- Angina.
- Stroke.
- High blood pressure (or hypertension).
- Heart failure.

Many things can put a person at risk for heart and cardiovascular disease. The more risk factors (or things that increase risk) a person has, the greater the chance that heart or cardiovascular disease will develop.

Factors include the following:

- age;
- smoking (active or passive);
- high blood pressure;
- high blood cholesterol;
- physical inactivity;
- excessive body weight; and
- diabetes.

There are some factors which cannot be controlled such as getting older, family health history, and race. However it is possible to control the three biggest risk factors for heart and cardiovascular disease - smoking, high blood pressure, and high blood cholesterol. Having a low saturated fat, low cholesterol diet and getting regular exercise are excellent health habits. These good health habits will lower blood pressure and keep blood sugar and blood cholesterol levels healthy.

Cholesterol is a fatty substance made by the liver and found in all parts of the body. The body uses cholesterol to produce cell membranes, hormones, vitamin D, and the bile acids that help to digest fat. It takes only a small amount of cholesterol in the blood to meet these needs, and the liver makes all the cholesterol the body needs.

Cholesterol is also accumulated from food. Eating too much cholesterol in animal foods like meats, whole milk dairy products, egg yolks, poultry, and fish can increase cholesterol levels. However, saturated fat in diets is the main culprit that causes cholesterol levels to rise.

Cholesterol travels through the blood in packages called lipoproteins. Low density lipoprotein (LDL) and high density lipoprotein (HDL) are two types of lipoproteins. LDL is often called the "bad" type of cholesterol because it can cause build up and blockage in the arteries that carry blood to the heart. HDL is known as "good" cholesterol because it helps remove cholesterol from the blood, preventing build up and blockage in the arteries.

Medicines used to lower cholesterol levels, if needed, are used along with lifestyle changes. The main goal of cholesterol-lowering treatment is to lower LDL (bad cholesterol) levels enough to reduce the risk of getting heart disease or having a heart attack. There are several types of drugs available for cholesterol lowering, including statins, bile acid sequestrants, nicotinic acid, and fibric acids. Each class of drugs has its own benefits, side effects and cautions.

| **Drug class** | **Generic and brand names** | **Benefits** | **Side effects and cautions*** |
|---|---|---|---|
| Statins | Atorvastatin (Lipitor)<br>Fluvastatin (Lescol)<br>Lovastatin (Altocor, Mevacor)<br>Pravastatin (Pravachol)<br>Rosuvastatin (Crestor)<br>Simvastatin (Zocor) | Reduce LDL and triglycerides, and moderately increase HDL | Upset stomach, gas, constipation, abdominal pain, cramps, muscle soreness, pain and weakness, increased blood levels of some statins with grapefruit juice consumption |
| Bile acid-binding resins | Cholestyramine (Questran)<br>Colesevelam (WelChol)<br>Colestipol (Colestid) | Reduce LDL | Constipation, bloating, nausea, gas |
| Cholesterol absorption inhibitors | Ezetimibe (Zetia) | Reduce LDL, slightly decrease triglycerides and slightly increase HDL | Stomach pain, fatigue |
| Combination cholesterol absorption inhibitor and statin | Ezetimibe/simvastatin (Vytorin) | Reduce LDL and triglycerides and moderately increase HDL | Same as statins and cholesterol absorption inhibitors |
| Fibrates | Fenofibrate (Lofibra, Tricor)<br>Gemfibrozil (Lopid) | Reduce triglycerides and modestly increase HDL | Gastrointestinal discomfort, increased risk of gallstones |
| Niacin (vitamin B-3, nicotinic acid) | A variety of prescription or over-the-counter preparations available in three forms: Immediate release, timed release, extended release | Increase HDL and reduce LDL and triglycerides | Flushing of face and neck, nausea, vomiting, diarrhoea, gout, high blood sugar, peptic ulcers |
| *All types of cholesterol-lowering drugs - with the possible exception of cholesterol absorption inhibitors - may cause liver function abnormalities. | | | |

European patent application no. EP 0 617 963 A discloses a lipid metabolism improving medicinal composition containing a phosphoric acid diester compound having a specific formula or a pharmacologically acceptable salt thereof.

Whilst these cholesterol lowering drugs are very important in reducing the risk of heart disease, there is the

downside with all of them that once the desired level has been achieved, it is necessary to continue taking the drugs indefinitely to maintain that level.

There is thus a need for a pharmaceutical substance which can be used to lower lipid levels which has fewer side effects than the current drugs and which does not lead to an indefinite need to administer the drug.

## Tocopherol

Low levels of α-tocopherol (vitamin E) have been associated with increased incidence of coronary heart disease. Conversely, increased intake of α-tocopherol has been shown to have protective effects against heart disease. Since vitamin E is an antioxidant, it is thought to target the cause of atherosclerosis by preventing oxidation of LDL. Studies have also been undertaken to examine potential non-antioxidant mechanisms of vitamin E which could prevent formation of atherosclerotic plaques. Such responses include inhibition of smooth muscle cell proliferation, preservation of endothelial function, inhibition of monocyte-endothelial adhesion, inhibition of monocyte reactive oxygen species and cytokine release, and inhibition of platelet adhesion and aggregation. Clinical trials with vitamin E have however been equivocal in demonstrating treatment of atherosclerosis. Current vitamin E supplements are therefore not a useful clinical option to combat atherosclerosis.

In international patent application no. WO 03/026673, there is disclosure that having increased storage levels of vitamins, including tocopheryl phosphate, could be beneficial in alleviating or treating inflammatory conditions such as coronary disease, atherosclerosis and diabetes. However, there is no disclosure of lowering the blood levels of lipids such as cholesterol. The process of inflammation involves a complicated set of pathways. These pathways are not involved in the pathways of lipid-metabolism, cholesterol uptake etc.

Tocopheryl phosphate has also been disclosed in international patent application no. WO 2004/064831 as having properties related to inhibiting the proliferation of monocytes/macrophages, proliferation of smooth muscle cells, the expression of CD36 receptors and the uptake of oxidized LDL. However, there is no disclosure of lowering the blood levels of lipids such as cholesterol. There are plenty of studies that have shown that CD36 promotes changes in response to proteins that accumulate in Alzheimer's disease and atherosclerosis. These processes have nothing to do with lipid metabolism. For example, drugs such as Malaria treatments and Alzheimers treatments are aimed at the CD36 expression but do not alter lipid profiles.

## Summary of the invention

It has now been surprisingly found that the phosphate derivatives of electron transfer agents are more effective than the non-phosphorylated electron transfer agents at lowering the blood levels of one or more of the following lipids:

- LDL cholesterol,
- triglycerides, and
- overall cholesterol.

In a first aspect, the present invention relates to one or more phosphate derivatives of one or more electron transfer agents for use in a method for lowering increased circulating blood levels of a lipid selected from the group consisting of LDL cholesterol, triglycerides, overall cholesterol and mixtures thereof, wherein the phosphate derivative of the electron transfer agent is selected from the group consisting of mono-tocopheryl phosphate, di-tocopheryl phosphate, metal salts thereof and mixtures thereof.

In a second aspect the present invention refers to the use of one or more phosphate derivatives of one or more electron transfer agents for the manufacture of a medicament for lowering increased circulating blood levels of a lipid selected from the group consisting of LDL cholesterol, triglycerides, overall cholesterol and mixtures thereof, wherein the phosphate derivative of the electron transfer agent is selected from the group consisting of mono-tocopheryl phosphate, di-tocopheryl phosphate, metal salts thereof and mixtures thereof.

A person skilled in the art will understand that the invention will be useful in relation to therapeutic treatment of diseases which are associated with increased blood levels of one or more of the following lipids: LDL cholesterol, triglycerides, and overall cholesterol. Examples of such diseases include cardiovascular disease, atherosclerosis, diabetes mellitus, chronic renal disease, primary and secondary hyperlipidemias and dyslipidemia, retinopathies, liver & spleen enlargement and xanthomas.

A pharmaceutical formulation comprising an effective amount of one or more phosphate derivatives of one or more electron transfer agents as defined above and optionally a suitable carrier or diluent can be administered to a subject having or at risk of developing the disease.

Preferably, the subject is an animal, more preferably the animal is a human.

The term "effective amount" is used herein to refer to an amount which is sufficient to lower increased circulating blood levels of one or more of the following lipids: LDL cholesterol, triglycerides, and overall cholesterol. A person skilled in the art will understand that this amount will vary from patient to patient and is usually determined from experience with clinical administration to particular patients.

"Electron transfer agents" are a class of chemicals which may be phosphorylated and which (in the non-phosphorylated form) can accept an electron to generate a relatively stable molecular radical or accept two electrons to allow the compound to participate in a reversible redox system. Examples of classes of electron transfer agent compounds that may be phosphorylated include hydroxy chromans including α-, β-, γ- and δ-tocols in enantiomeric and racemic forms; quinols being the reduced forms of vitamin K1 and ubiquinone; hydroxy carotenoids including retinol; calciferol and ascorbic acid. Examples of the electron transfer agent include tocopherol and other tocols, retinol, vitamin K1 and mixtures thereof.

The tocols include all isomers of derivatives of 6:hydroxy 2:methyl chroman (see structure below) where $R_1$, $R_2$ and $R_3$ may be hydrogen or methyl groups, that is, the α-5:7:8 tri-methyl; β-5:8 di-methyl; γ-7:8 di-methyl; and δ-8 methyl derivatives. In the tocopherols, $R_4$ is substituted by 4:8:12 tri-methyl tridecyl and includes various stereoisomers and optical isomers (chiral centres are indicted by the *). In the tocotrienols, $R_4$ is substituted by 4:8:12 tri-methyl trideca-3:7:11 triene and the 2 position may be stereoactive as R or S stereoisomers. Examples of tocotrienols include α-tocotrienol, δ-tocotrienol, γ-tocotrienol and mixtures thereof.
Tocopherol is used as an electron transfer agent in the form of mono-tocopheryl phosphate, di-tocopheryl phosphate, metal salts thereof and mixtures thereof in the present invention.

Most preferably, the electron transfer agent is selected from the group consisting of α-tocopherol, δ-tocopherol, γ-tocopherol and mixtures thereof.

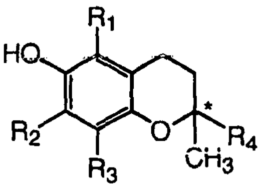

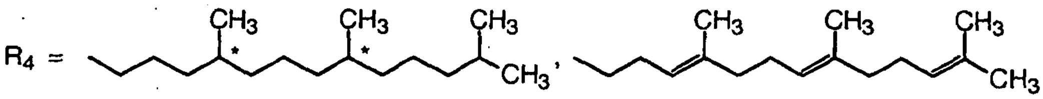

"Phosphate derivatives" are compounds covalently bound by means of an oxygen to the phosphorus atom of a phosphate group thus forming a carbon-oxygen -phosphorous bond. The oxygen atom is typically derived from a hydroxyl group on the electron transfer agent. The term includes the acid forms of phosphorylated electron transfer agents, salts of the phosphates including metal salts such as sodium, magnesium, potassium and calcium and any other derivative where the phosphate proton is replaced by other substituents such as ethyl or methyl groups or phosphatidyl groups. The term includes mixtures of phosphate derivatives, especially those which result from phosphorylation reactions, as well as each of the phosphate derivatives alone. For example, the term includes a mixture of mono-tocopheryl phosphate (TP) and di-tocopheryl phosphate (T2P) as well as each of TP and T2P alone. Suitable mixtures are described in international patent application no. PCT/AU01/01475.

Phosphates and metal salts thereof are used as phosphate derivatives in the form of mono-tocopheryl phosphate, di-tocopheryl phosphate, metal salts thereof and mixtures thereof in the present invention.

In the present invention, the one or more phosphate derivatives of one or more electron transfer agents is preferably selected from the group consisting of mono-tocopheryl phosphate, di-tocopheryl phosphate, and mixtures thereof. In one preferred embodiment, the one or more phosphate derivatives of one or more electron transfer agents is a mixture of one or more of mono-tocopheryl phosphate, and di-tocopheryl phosphate.

The use a phosphate derivative such as a phosphatide is disclosed for some situations where additional properties such as increased water solubility are preferred. Phosphatidyl derivatives are amino alkyl derivatives of organic phosphates. These derivatives may be prepared from amines having a structure of $R_1R_2N(CH_2)_nOH$ wherein n is an integer between 1 and 6 and $R_1$ and $R_2$ may be either H or short alkyl chains with 3 or less carbons. $R_1$ and $R_2$ may be the same or different. The phosphatidyl derivatives are prepared by displacing the hydroxyl proton of the electron transfer agent with a phosphate entity that is then reacted with an amine, such as ethanolamine or N,N'-dimethylethanolamine, to generate the phosphatidyl derivative of the electron transfer agent. One therapy of preparation of the phosphatidyl

derivatives uses a basic solvent such as pyridine or triethylamine with phosphorous oxychloride to prepare the intermediate which is then reacted with the hydroxy group of the amine to produce the corresponding phosphatidyl derivative, such as P cholyl P tocopheryl dihydrogen phosphate.

Complexes of phosphate derivatives of the electron transfer agents are also disclosed for use in some situations where additional properties such as improved stability or deliverability may be useful. The term "complexes of phosphate derivatives" refers to the reaction product of one or more phosphate derivatives of electron transfer agents with one or more complexing agents selected from the group consisting of amphoteric surfactants, cationic surfactants, amino acids having nitrogen functional groups and proteins rich in these amino acids as disclosed in international patent application no. PCT/AU01/01476. Examples of proteins rich in these amino acids are those proteins having either at least I in 62 amino acids as arginine, or at least 1 in 83 histidine, or at least 1 in 65 as lysine, such as the various forms of the protein casein. Other examples include insulin, parathyroid hormone (PTH), glucagon, calcitonin, adrenocorticotropic hormone (ACTH), prolactin, interferon-$\alpha$ and -$\beta$ and -$\gamma$, leutenising hormone (LH) (also known as gonadotropin releasing hormone), follicle stimulating hormone (FSH) and colony stimulating factor (CSF). The amino acid composition of most of these examples is listed in the table.

| **Amino acids in protein** | **Amino acids** | **Ratio of Total Amino acids** |
|---|---|---|
| **Insulin** | **110** | |
| arg | 5 | 1 in 22 |
| his | 2 | 1 in 55 |
| lys | 2 | 1 in 55 |
| | | |
| PTH | **84** | |
| arg | 5 | 1 in 17 |
| his | 0 | 0 |
| lys | 5 | 1 in 17 |
| | | |
| **Glucagon** | **180** | |
| arg | 16 | 1 in 11 |
| his | 4 | 1 in 45 |
| lys | 10 | 1 in 18 |
| | | |
| **Calcitonin** | **93** | |
| arg | 6 | 1 in 16 |
| his | 3 | 1 in 31 |
| lys | 5 | 1 in 19 |
| | | |
| **ACTH** | **41** | |
| arg | 3 | 1 in 14 |
| his | 1 | 1 in 41 |
| lys | 4 | 1 in 10 |
| | | |
| **Prolactin** | **220** | |
| arg | 12 | 1 in 18 |
| his | 9 | 1 in 13 |
| lys | 11 | 1 in 11 |
| | | |
| **Interferon-alpha and beta** | **133** | |
| arg | 7 | 1 in 19 |
| his | 2 | 1 in 83 |
| lys | 7 | 1 in 19 |
| | | |
| **Interferon -gamma** | **166** | |

(continued)

| Amino acids in protein | Amino acids | Ratio of Total Amino acids |
|---|---|---|
| arg | 8 | 1 in 21 |
| his | 2 | 1 in 83 |
| lys | 21 | 1 in 8 |
| | | |
| LH | **92** | |
| arg | 5 | 1 in 18 |
| his | 2 | 1 in 46 |
| lys | 7 | 1 in 13 |
| | | |
| FSH | **129** | |
| arg | 5 | 1 in 26 |
| his | 2 | 1 in 65 |
| lys | 9 | 1 in 14 |
| | | |
| CSF | **144** | |
| arg | 6 | 1 in 24 |
| his | 3 | 1 in 48 |
| lys | 6 | 1 in 24 |
| | | |
| **GH domain AOD9604** | **16** | |
| arg | 2 | 1 in 8 |

The disclosed complexing agents are selected from the group consisting of arginine, lysine and tertiary substituted amines, such as those according to the following formula:

$$NR^1R^2R^3$$

wherein $R^1$ is chosen from the group comprising straight or branched chain mixed alkyl radicals from C6 to C22 and carbonyl derivatives thereof;
$R^2$ and $R^3$ are chosen independently from the group comprising H, $CH_2COOX$, $CH_2CHOHCH_2SO_3X$, $CH_2CHOHCH_2OPO_3X$, $CH_2CH_2COOX$, $CH_2COOX$, $CH_2CH_2CHOHCH_2SO_3X$ or $CH_2CH_2CHOHCH_2OPO_3X$ and X is H, Na, K or alkanolamine provided $R^2$ and $R^3$ are not both H; and
wherein when $R^1$ is RCO then $R^2$ may be $CH_3$ and $R_3$ may be $(CH_2CH_2)N(C_2H_4OH)$-$H_2CHOPO_3$ or $R^2$ and $R^3$ together may be $N(CH_2)_2N(C_2H_4OH)CH_2COO$-.

Disclosed complexing agents include arginine, lysine or lauryliminodipropionic acid where complexation occurs between the alkaline nitrogen centre and the phosphoric acid ester to form a stable complex.

The phosphate derivative of the electron transfer agent may be administered to humans or animals through a variety of dose forms such as supplements, enteral feeds, parenteral dose forms, suppositories, oral dose forms, pulmonary and nasal delivery forms, dermal delivery including patches and creams.

For example, the phosphate derivative of the electron transfer agent may be administered by an orally or parenterally administered dose form. These include tablets, powders, chewable tablets, capsules, oral suspensions, suspensions, emulsions or fluids, children's formulations, enteral feeds, nutraceuticals, and functional foods.

The dose form may further include any additives routinely used in preparation of that dose form such as starch or polymeric binders, sweeteners, coloring agents, emulsifiers, and coatings. Other suitable additives will be readily apparent to those skilled in the art.

In one embodiment, the dose form has an enteric coating as disclosed in international patent application PCT/AU01/01206.

In another embodiment, the dose form is a topical formulation as disclosed in international patent application PCT/AU02/01003.

The dose form may contain other pharmaceutical compounds which do not antagonise the activity of the phosphate derivatives of electron transfer agents. The other pharmaceutical compound may be administered before, with or after the one or more phosphate derivatives of one or more electron transfer agents. Preferably, the other pharmaceutical compounds are drugs for heart and cardiovascular disease and hypercholesterolaemic or dislipidaemic

compounds. More preferably, the other pharmaceutical compounds are selected from the group consisting of cholesterol absorption inhibitors such as ezetimibe, cholesterol ester transfer protein inhibitors such as torcetrapib, other HDL increasing pharmaceutical compounds, statins, phosphate derivatives of statins and mixtures thereof. Examples of appropriate statins include provastatin, lovastatin and atorvastatin and phosphates thereof.

Preferably, the subject is an animal. More preferably, the animal is a mammal. Most preferably, the mammal is a human.

## Drawings

Various embodiments/aspects of the invention will now be described with reference to the following drawings in which:

Figure 1 is shows the results of Example 1 at 2 weeks.

Figure 2 shows the results of Example I at 4 weeks.

Figure 3 shows the results from Example 2.

## Examples

Various embodiments/aspects of the invention will now be described with reference to the following examples.

### Example 1

This example evaluates the potential anti-CVD effects of a tocopheryl phosphate mixture in a well accepted CVD mouse model, the apolipoprotein E (APOE) mouse. The anti-CVD effects are assessed by decreases in the elevated plasma cholesterol, triglyceride and LDL levels.

The APOE knockout mouse model has been widely used in cardiovascular research as it mimics many of the properties observed clinically as part of the human disease. The APOE knockout mouse displays elevated circulating lipid levels from about 6 months of age. Placing these animals on a high fat, high cholesterol diet (i.e. 21 % fat, 0.15% cholesterol) exacerbates the CVD, and therefore these symptoms are observed sooner.

Methods

*Animals:* Male APOE knockout mice (15 - 20g) were obtained from the Animal Resource Centre, Perth, Australia. They were fed a vitamin E stripped diet that contained 21 % fat and 0.15% cholesterol rodent pellets from Glen Forrest Stockfeeders, W.A., Australia. The mice were housed in standard laboratory cages with natural lighting, and acclimatised for at least 7 days before use.

In the current study the APOE mice were placed on a high fat, high cholesterol diet for a total of 8 weeks. Four weeks into this diet the animals are treated daily, via oral gavage, with either vehicle (1% CMC), tocopherol acetate (TA) at 100 mg/kg, or TP mixture (TPm) at 33.25, 66.5 or 133 mg/kg. The assessment of any improvement in the CDV of these mice involved blood being taken at regular intervals during the treatment, to assess the lipid levels and sectioning of the aortic arch, for assessment of the plaque formation at the end of the treatment period.

*Reagents:*

Tocopherol Acetate (***TA***) (Sigma catalogue no.T-3001)

Tocopheryl phosphate mixture (***TPm***) containing mono-tocopheryl phosphate and di-tocopheryl phosphate in a ratio of 2:1 (made in house batch no. SGNaTPm/21-10-04)

Carboxymethylcellulose (Sigma catalogue no. C-5678)

Milli Q water (in-house supply)

*Formulation Preparation:* Solutions of TPm and TA were prepared in 1% carboxymethylcellulose (CMC) at the following concentrations: for TA, 15 mg/ml (reference example), and for TPm, 4.99, 9.98 and 19.95 mg/ml (examples according to the invention) for dosing mice at TA 100 mg/kg and TPm at 33.25, 66.5 and 133 mg/kg, respectively. In preparing these solutions, the appropriate amount of each compound was made up in the 1% CMC and then placed in a water bath sonicator with warm water (about 50°C) for 15 minutes. (The TPm dose of 133 mg/kg is used as the TA 100 mg/kg equivalent dose. Therefore the subsequent 66.5 and 33.25 mg/kg doses are 50 and 25% equivalent TA 100 mg/kg doses, respectively).

*Dosing:* Mice were weighed weekly, and the doses of each compound were calculated based on this weight for that dosing week. The animals were dosed between 7:30 am-11:00 am, each morning of the treatment period with a stainless steel gavage needle.

*Blood collection:* On 3 occasions the mice were restrained firmly and the tail nicked and about 50 μl of blood was collected into Capiject™ tubes. The tubes were then centrifuged at 8,000 x g and the plasma collected, for lipid analysis (i.e. total Cholesterol, triglyceride, HDL and LDL measurements, carried out by Gribbles Pathology).

The mice were bled prior to the commencement of the study (pre-bleeds), at the end of 4 weeks on the respective diets (prior to the commencement of the compound treatments) and 2 weeks into the treatment period. At the end of the treatment period, blood was collected from the animals directly from the heart after having been sacrificed by $CO_2$ asphyxiation. The APOE mice placed on the high fat and high cholesterol diet, increased 2-3 fold their plasma cholesterol, LDL and triglyceride levels during this feeding period and 2 weeks into the treatment period. At the end of the treatment period, blood was collected from the animals directly from the heart after having been sacrificed by $CO_2$ asphyxiation. The APOE mice placed on the high fat and high cholesterol diet, increased 2-3 fold their plasma cholesterol, LDL and triglyceride levels during this feeding period This level was considered very high, as these mice already have quite elevated fasting lipid levels without being placed on an atherogenic diet. This was considered a good starting point in assessing the effectiveness of TPm to reduce these elevated lipid levels.

*Analysis of plasma triglyceride:* Measurement of plasma triglyceride took place with the use of the Triglyceride Determination Kit (Sigma, Catalogue No. TR0100). The procedure involves enzymatic hydrolysis by lipase of the triglycerides to glycerol and free fatty acids. The glycerol produced is then measured by coupled enzyme reactions shown below:

| | |
|---|---|
| Lipoprotein lipase | Triglycerides -----------------> Glycerol + Fatty Acids |
| Glycerol kinase | Glycerol + ATP > Glycerol-1-phosphate + ADP |
| Glycerol phosphate oxidase | Glycerol-1-phosphate -----------------> Dihydroxyacetone phosphate + $H_2O_2$ |
| Peroxidase | $H_2O_2$ + 4-Amino antipyrine + Sodium N-ethyl-N-(3-sulfopropyl) m-anisidine -----------------> Quinoneimine Dye + $H_2O$ |

The reagents in the kit are prepared as per the manufacturers directions. The free glycerol standard reagent and samples are warmed to room temperature. A set of cuvets are prepared for Blank, Standard and Samples. 0.8ml of Free Glycerol reagent is added to each cuvet, followed by 10μl of water, glycerol standard or plasma, respectively. The samples are mixed, by inversion, and incubated at 37°C for 5 minutes. The absorbance is then read at 540nm and recorded as initial absorbance (IA). 0.2ml of triglyceride reagent is then added to each cuvet and they are again mixed by inversion, and incubated for a further 5 minutes at 37°C. The final absorbance (FA) is then read and recorded at 540nm. The total triglyceride concentration in plasma is then calculated are follows:

$$\text{Total triglyceride} = \frac{(FA_{sample} - FA_{blank})}{(FA_{standard} - FA_{blank})} * \text{Concentration of standard}$$

*Analysis of plasma cholesterol:* Measurement of plasma cholesterol took place with the use of the Infinity™ Cholesterol Reagent Kit (Thermo Electron Corp., Catalogue No. TR13521). The reagent is based on the following reactions:

| | |
|---|---|
| Cholesterol esterase | Cholesterol Esters -----------------> Cholesterol + Fatty Acids |
| Cholesterol oxidase | Cholesterol + $O_2$ -----------------> Cholest-4-en-one + $H_2O_2$ |
| Peroxidase | $2H_2O_2$ + Hydroxybenzoic acid + 4-Aminoantipyrine -----------------> Quinoneimine Dye + $4H_2O$ |

(1). Cholesterol esters are enzymatically hydrolysed by cholesterol esterase to cholesterol and free fatty acids.

(2). Free cholesterol, including that originally present, is then oxidized by cholesterol oxidase to cholest-4-en-3-one and hydrogen peroxide.

(3). The hydrogen peroxide combines with HBA and 4-aminoantipyrine to form a chromophore (quinoneimine dye)

which may be quantitated at 500-550nm.

The plasma is incubated with Cholesterol Reagent™ (1:100). For example a sample volume of 3μl is incubated with 300μl of Cholesterol Reagent™, in a microtitre plate and incubated at 37°C for 5 minutes. This is conducted for a calibrator also supplied in the kit.

The total cholesterol is then calculated as follows:

$$\text{Cholesterol} = \quad \Delta\, \frac{\Delta\text{Abs}/\text{min of unknown}}{\text{Abs}/\text{min of calibrator}} * \text{Calibrator concentration}$$

Example:

Absorbance of calibrator = 0.35

Absorbance of unknown = 0.25

Value of calibrator = 7.0 mmol/L

Cholesterol = 0.25/0.35 X 7.0 = 5.0 mmol/L

*Analysis of plasma HDL:* Measurement of HDL took place with the use of the Infinity™ HDL Cholesterol Reagent Kit (Thermo Electron Corp., Catalogue No. TR39601).

The plasma (4μl) is placed in a microtitre plate, and is incubated at 37°C for 5 minutes with 300μl of Reagent 1, followed by further 3 minute incubation after the addition of 100μl of Reagent 2. The absorbance is then read at 600nm. As for the cholesterol kit a calibrator also supplied in the kit is used also for the calculation.

*Analysis of plasma LDL:* Measurement of LDL took place with the use of the Infinity™ LDL Cholesterol Plus Reagent Kit (Thermo Electron Corp., Catalogue No. 3365-030).

The plasma (4μl) is placed in a microtitre plate, and is incubated at 37°C for 5 minutes with 300μl of Reagent 1, followed by further 5 minute incubation after the addition of 100μl of Reagent 2. The absorbance is then read at 600nm. As for the cholesterol kit a calibrator also supplied in the kit is used also for the calculation.

*Statistical Analysis:* Results are expressed as mean ± SD. A Student's t-test was performed to determine whether there were significant differences in TA or TPm treated mice (whether it is cholesterol, triglyceride, HDL, LDL or plaque size) compared to no treatment or vehicle control groups. For a study of this type $P<0.05$, (*) was considered significant.

<u>Results and Discussion</u>

Figure 1 shows the results obtained after 2 weeks of treatment.

Figure 2 shows the results obtained after 4 weeks of treatment.

The administration of TPm, in particular at 33.25 mg/kg, gave a significant decrease in plasma total cholesterol and LDL concentrations compared to no treatment or vehicle alone treated mice, after 2 weeks of treatment. Following the 4 weeks of treatment, the 33.25 mg/kg dose of TPm still provided a significant decrease in plasma triglyceride levels compared to no treatment or vehicle alone controls. These results suggest that TPm (in particular at 33.25 mg/kg) is potentially effective in lowering elevated cholesterol, triglyceride and LDL levels circulating in blood.

Example 2

This example evaluated the effects of a tocopheryl phosphate mixture (TPm) (mono-tocopheryl phosphate and di-tocopheryl phosphate) on the development of atherosclerotic lesions in male APOE deficient mice.

Methodology

Twenty-eight mice were divided into 4 groups: 2 control groups, a tocopherol acetate (TA) group (150mg TA/kg feed; reference example) and a TPm group (200mg TPm/kg the feed containing 7% fat; example according to the invention).

***Diets:***

'Induction phase' - The induction phase consisted of the first 16 weeks of the treatment period. During this period the animals were fed a mouse pellet diet low in vitamin E (containing less than 20 mg vitamin E per kg food, with a 7% total fat; modified version of the standard AIN93G rodent diet (SF05-040, Specialty feeds, Glen Forrest, WA Australia). Control animals were fed the diet alone, while TA-feed contained 150mg TA/kg feed and TPm-feed contained 200mg TPm/kg feed. These feeds delivered on average doses of 21 and 26 mg/kg body weight, respectively. The 26mg/kg TPm dose was calculated to be the tocopherol equivalence of the TA dose.

'Challenge phase' - This phase consisted of the final 8 weeks of the treatment period. During this period the animals were fed a low vitamin E, high fat (21%), high cholesterol (0.15%) specialised rodent pellet diet (HFHC; SF04-055 mouse diet is a version of the standard SF00-219 diet containing less than 20mg vitamin E per kg; Specialty feeds, Glen Forrest, WA, Australia).

The 4 groups of animals were placed on the diet regimes outlined in the table below:

| Treatment Group | Diet for weeks 0-16 | Diet for weeks 16-24 |
|---|---|---|
| **Control (C24)** | SF05-040 | SF05-040 |
| **Control (C16/8)** | SF05-040 | SF04-055 |
| **TA** | SF05-040 + TA (150mg TA/kg feed) | SF04-055 + TA (150mgTA/kg feed) |
| **TPm** | SF05-040 + TPm (200mg TPm/kg feed) | SF04-055 + TPm (200mg TPm/kg) |

Of the two control animal groups only one was placed on the SF04-055, HFHC diet, while the other control group was maintained on the SF05-040 mouse pellet diet (only 7% fat) for the entire treatment period 24 week treatment period (C24). This was done so as to establish the effect of the HFHC diet alone on the various atherosclerotic parameters being measured, and to assess whether or not treatments with the various compounds were as good as animals maintained on normal diets. TA treated mice were fed pellets with 150mg TA/kg feed and TPm treated mice were fed the pellets with 200mg TPm/kg feed. These feeds delivered doses averaging 21 and 26 mg/kg body weight, respectively. The 26 mg/kg TPm dose was calculated to be the tocopherol equivalent to the TA dose.

During the induction phase, the control mice developed mild hypercholesterolemia and atherosclerotic lesions. After 16 weeks of treatment with TPm, the mice showed a 34% reduction in total cholesterol (11.44 +/- 1.37 vs 17.38 +/- 1.47 mmol/L), 51% reduction in triglycerides (0.99 +/- 0.14 vs 2.00 +/- 0.58 mmol/L) and a 44% reduction in LDL-C (4.67 +/- 0.70 vs 8.38 +/- 0.76 mmol/L) compared to control animals. These reductions were significantly different from control animals, and were far greater than those seen with TA treatment.

After the challenge phase, the control mice developed severe hypercholesterolemia and advanced atherosclerotic lesions. The TA-treated mice showed no significant reduction in plasma lipid levels or evidence for lesion regression; although there was an average 12% decrease in lesion area (this was not significant). However, the TPm treatment gave a reduction of 15% in total cholesterol (43.8 +/- 4.38 vs 37.08 +/- 5.15 mmol/L), 28% reduction in triglycerides (1.63 +/- 0.22 vs 2.27 +/- 0.20 mmol/L) and 16% reduction in LDL-C (15.02 +/- 2.61 vs 17.95 +/- 1.51 mmol/L) as well as a significant reduction (58%) in aortic lesion formation.

**Table 1.** Mouse total cholesterol level comparisons (mean ± SD; mmol/L) during the induction and challenge phases of the study.

| Group (n) | Baseline | Induction phase | Challenge phase |
|---|---|---|---|
| **Week** | **0** | **16** | **24** |
| Control SF05-040 diet alone (n=8-12) | 11.96 ± 0.82 (n=12) | 13.43 ± 2.21 (n=12) | 11.43 ± 1.89 (n=8) |
| Control (n=8) | 12.80 ± 1.35 | 17.38 ± 1.47 | 43.8 ± 4.38# |
| TA (n=8) | 12.75 ± 1.41 | 14.47 ± 1.32 | 47.13 ± 4.44 |

(continued)

| Group (n) | Baseline | Induction phase | Challenge phase |
|---|---|---|---|
| Week | 0 | 16 | 24 |
| TP (n=8) | 11.61 ± 1.24 | 11.44 ± 1.37* | 37.08 ± 5.15* |
| # indicates control mice maintained on HFHC diet (for the final 8 weeks of the treatment) had significantly higher plasma cholesterol levels to control mice on the normal diet (P<0.05). * indicates significance (P<0.05) from Control animals. | | | |

**Table 2.** Mouse triglyceride level comparisons (mean ± SD; mmol/L) during the induction and challenge phases of the study.

| Group (n) | Baseline | Induction Phase | Challenge Phase |
|---|---|---|---|
| Week | 0 | 16 | 24 |
| Control SF05-040 diet alone (n=8-12) | 1.73 ± 0.13 (n=12) | 1.10 ± 0.25 (n=12) | 1.45 ± 0.21 (n=8) |
| Control (n=8) | 1.18 ± 0.28 | 2.00 ± 0.58 | 2.27 ± 0.20# |
| TA (n=8) | 1.75 ± 0.25 | 1.30 ± 0.22* | 2.23 ± 0.29 |
| TP (n=8) | 1.54 ± 0.38 | 0.99 ± 0.14* | 1.63 ± 0.22* |
| # indicates control mice maintained on HFHC diet (for the final 8 weeks of the treatment) had significantly higher plasma cholesterol levels to control mice on the normal diet (P<0.05). * indicates significance (P<0.05) from Control animals. | | | |

**Table 3.** Mouse LDL-C level comparisons (mean ± SD; mmol/L) during the induction and challenge phases of the study.

| Group (n) | Baseline | Induction Phase | Challenge Phase |
|---|---|---|---|
| Week | 0 | 16 | 24 |
| Control SF05-040 diet alone (n=8-12) | 6.83 ± 0.54 (n=12) | 7.65 ± 0.54 (n=12) | 5.20 ± 0.96 (n=8) |
| Control (n=8) | 8.08 ± 1.20 | 8.38 ± 0.76 | 17.95 ± 1.51# |
| TA (n=8) | 8.85 ± 1.66 | 6.51 ± 0.71 | 18.98 ± 2.29 |
| TP (n=8) | 7.84 ± 1.51 | 4.67 ± 0.70* | 15.02 ± 2.61 |
| # indicates control mice maintained on HFHC diet (for the final 8 weeks of the treatment) had significantly higher plasma cholesterol levels to control mice on the normal diet (P<0.05). * indicates significance (P<0.05) from Control animals. | | | |

**Table 4.** Atheromatous lesion area (mean ± SD, % lesion coverage), at the end of the treatment period.

| Group (n) | % Lesion coverage |
|---|---|
| Control SF05-040 diet alone (n=8) | 8.9 ± 1.7 |
| Control C16/8 (n=8) | 10.7 ± 1.3 |
| TA (n=8) | 9.4 ± 1.1 |
| TPm (n=8) | 4.5 ± 1.3* |
| * indicates significance (P<0.05) from Control animals. | |

Figure 3 shows the aortic lesion formation assessment of aortae by Oil red O staining.

The aortic root, thoracic and abdominal aortae were stained with Oil red O (ORO), (which stains lipids red), showed substantial lipid deposits in vascular atherosclerotic lesions. Table 4 shows the lesion sizes at the end of the 24 week treatment period across each of the treatment groups. On average at the end of the induction period (at week 16) each mouse had approximately 5% atheromatous lesions coverage per aortic region, *(data not shown).* The lesion area was increased to 8.9% by the end of the 24 week period in animals maintained on the induction diet alone (7% fat alone). Animals that were placed on the atherogenic diet for the final 8 weeks showed on average of 10.7% atheromatous lesions per aortic region compared to 9.4% in TA and 4.5% in TPm treated mice. TA treatment saw a 12% improvement in atherosclerotic lesions (this was not statistically significant), while TPm treatment saw a significant 58% reduction in lesion formation compared to control mice maintained on the same diet regime.

Conclusion

The findings show a significant reduction in the lipid profiles (LDL, total cholesterol and triglyceride) in animals treated with TPm, indicating that TPm treatment may treat hyperdyslipidemia and related diseases. As a secondary outcome, the findings show a significant decrease in the atherosclerotic lesion size in TPm treated APO E-deficient mice, indicating that TPm treatment may treat or slow the progression of atherosclerotic lesions in this mouse strain.

## Claims

1. One or more phosphate derivatives of one or more electron transfer agents for use in a method for lowering increased circulating blood levels of a lipid selected from the group consisting of LDL cholesterol, triglycerides, overall cholesterol and mixtures thereof, wherein the phosphate derivative of the electron transfer agent is selected from the group consisting of mono-tocopheryl phosphate, di-tocopheryl phosphate, metal salts thereof and mixtures thereof.

2. Use of one or more phosphate derivatives of one or more electron transfer agents for the manufacture of a medicament for lowering increased circulating blood levels of a lipid selected from the group consisting of LDL cholesterol, triglycerides, overall cholesterol and mixtures thereof, wherein the phosphate derivative of the electron transfer agent is selected from the group consisting of mono-tocopheryl phosphate, di-tocopheryl phosphate, metal salts thereof and mixtures thereof.

3. The phosphate derivatives for use in a method for lowering increased circulating blood levels of a lipid according to claim 1 or the use according to claim 2, wherein the electron transfer agent is selected from the group consisting of $\alpha$-tocopherol, $\delta$-tocopherol, $\gamma$-tocopherol, and mixtures thereof.

4. The phosphate derivatives for use in a method for lowering increased circulating blood levels of a lipid according to claim 1 or 3 or the use according to claim 2 or 3, wherein the one or more phosphate derivatives of one or more electron transfer agents is a mixture of mono-tocopheryl phosphate and di-tocopheryl phosphate.

5. The phosphate derivatives for use in a method for lowering increased circulating blood levels of a lipid according to claim 4 or use according to claim 4, wherein one or more other pharmaceutical compounds selected from the group consisting of cholesterol absorption inhibitors, cholesterol ester transfer protein inhibitors, HDL increasing pharmaceutical compounds, statins, their phosphate derivatives, and mixtures thereof are also to be administered.

6. The phosphate derivatives for use in a method for lowering increased circulating blood levels of a lipid according to any of claims 1, or 3 to 5 or the use according to any of claims 2 to 5, wherein the lowering increased circulating blood levels of a lipid alleviates symptoms, treats or prevents a disease selected from the group consisting of cardiovascular disease, atherosclerosis, diabetes mellitus, chronic renal disease, primary and secondary hyperlipidemias and dyslipidemia, retinopathies, liver and spleen enlargement, xanthomas and combinations thereof.

7. The phosphate derivatives for use in a method for lowering increased circulating blood levels of a lipid according to claim 6 or the use according to claim 6, wherein the disease is selected from the group consisting of chronic renal disease, primary and secondary hyperlipidemias and dyslipidemia, retinopathies, liver and spleen enlargement, xanthomas and combinations thereof.

## Patentansprüche

1. Ein oder mehrere Phosphatderivate eines oder mehrerer Elektronenübertragungsmittel zur Verwendung in einem Verfahren zur Senkung erhöhter Konzentrationen eines Lipids in zirkulierendem Blut, wobei das Lipid aus der Gruppe bestehend aus LDL-Cholesterin, Triglyceriden, Gesamtcholesterin und Gemischen davon ausgewählt ist, wobei das Phosphatderivat des Elektronenübertragungsmittels aus der Gruppe bestehend aus Monotocopherylphosphat, Ditocopherylphosphat, Metallsalzen davon und Gemischen davon ausgewählt ist.

2. Verwendung eines oder mehrerer Phosphatderivate eines oder mehrerer Elektronenübertragungsmittel zur Herstellung eines Medikaments zur Senkung erhöhter Konzentrationen eines Lipids in zirkulierendem Blut, wobei das Lipid aus der Gruppe bestehend aus LDL-Cholesterin, Triglyceriden, Gesamtcholesterin und Gemischen davon ausgewählt ist, wobei das Phosphatderivat des Elektronenübertragungsmittels aus der Gruppe bestehend aus Monotocopherylphosphat, Ditocopherylphosphat, Metallsalzen davon und Gemischen davon ausgewählt ist.

3. Die Phosphatderivate zur Verwendung in einem Verfahren zur Senkung erhöhter Konzentrationen eines Lipids in zirkulierendem Blut nach Anspruch 1 oder die Verwendung nach Anspruch 2, wobei das Elektronenübertragungsmittel aus der Gruppe bestehend aus $\alpha$-Tocopherol, $\delta$-Tocopherol, $\gamma$-Tocopherol und Gemischen davon ausgewählt ist.

4. Die Phosphatderivate zur Verwendung in einem Verfahren zur Senkung erhöhter Konzentrationen eines Lipids in zirkulierendem Blut nach Anspruch 1 oder 3 oder die Verwendung nach Anspruch 2 oder 3, wobei das eine oder die mehreren Phosphatderivate eines oder mehrerer Elektronenübertragungsmittel ein Gemisch von Monotocopherylphosphat und Ditocopherylphosphat ist.

5. Die Phosphatderivate zur Verwendung in einem Verfahren zur Senkung erhöhter Konzentrationen eines Lipids in zirkulierendem Blut nach Anspruch 4 oder Verwendung nach Anspruch 4, wobei eine oder mehrere andere pharmazeutische Verbindungen, ausgewählt aus der Gruppe bestehend aus Cholesterinabsorptionshemmern, Cholesterinester-Transferprotein-Hemmern, HDL-erhöhenden pharmazeutischen Verbindungen, Statinen, ihren Phosphatderivaten und Gemischen davon, ebenfalls zu verabreichen sind.

6. Die Phosphatderivate zur Verwendung in einem Verfahren zur Senkung erhöhter Konzentrationen eines Lipids in zirkulierendem Blut nach einem der Ansprüche 1 oder 3 bis 5 oder die Verwendung nach einem der Ansprüche 2 bis 5, wobei die Senkung erhöhter Konzentrationen eines Lipids in zirkulierendem Blut Symptome einer Erkrankung, ausgewählt aus der Gruppe bestehend aus kardiovaskulärer Erkrankung, Atherosklerose, Diabetes mellitus, chronischer Nierenkrankheit, primären und sekundären Hyperlipidämien und Dyslipidämie, Retinopathien, Leber- und Milzvergrößerung, Xanthomen und Kombinationen davon, lindert, diese behandelt oder diesen vorbeugt.

7. Die Phosphatderivate zur Verwendung in einem Verfahren zur Senkung erhöhter Konzentrationen eines Lipids in zirkulierendem Blut nach Anspruch 6 oder die Verwendung nach Anspruch 6, wobei die Erkrankung aus der Gruppe bestehend aus chronischer Nierenkrankheit, primären und sekundären Hyperlipidämien und Dyslipidämie, Retinopathien, Leber- und Milzvergrößerung, Xanthomen und Kombinationen davon ausgewählt ist.

## Revendications

1. Un ou plusieurs dérivés phosphates d'un ou plusieurs agents de transfert d'électrons destinés à être utilisés dans un procédé pour abaisser des taux accrus d'un lipide dans le sang circulant, ledit lipide étant choisi dans le groupe consistant en le LDL cholestérol, les triglycérides, le cholestérol total et leurs mélanges, où le dérivé phosphate de l'agent de transfert d'électrons est choisi dans le groupe consistant en le phosphate de mono-tocophéryle, le phosphate de di-tocophéryle, leurs sels métalliques et leurs mélanges.

2. Utilisation d'un ou plusieurs dérivés phosphates d'un ou plusieurs agents de transfert d'électrons pour la fabrication d'un médicament pour abaisser des taux accrus d'un lipide dans le sang circulant, ledit lipide étant choisi dans le groupe consistant en le LDL cholestérol, les triglycérides, le cholestérol total et leurs mélanges, où le dérivé phosphate de l'agent de transfert d'électrons est choisi dans le groupe consistant en le phosphate de mono-tocophéryle, le phosphate de di-tocophéryle, leurs sels métalliques et leurs mélanges.

3. Dérivés phosphates destinés à être utilisés dans un procédé pour abaisser des taux accrus d'un lipide dans le sang

circulant selon la revendication 1 ou utilisation selon la revendication 2, où l'agent de transfert d'électrons est choisi dans le groupe consistant en l'α-tocophérol, le δ-tocophérol, le γ-tocophérol et leurs mélanges.

4. Dérivés phosphates destinés à être utilisés dans un procédé pour abaisser des taux accrus d'un lipide dans le sang circulant selon la revendication 1 ou 3 ou utilisation selon la revendication 2 ou 3, où le un ou plusieurs dérivés phosphates d'un ou plusieurs agents de transfert d'électrons est un mélange de phosphate de mono-tocophéryle et de phosphate de di-tocophéryle.

5. Dérivés phosphates destinés à être utilisés dans un procédé pour abaisser des taux accrus d'un lipide dans le sang circulant selon la revendication 4 ou utilisation selon la revendication 4, où un ou plusieurs autres composés pharmaceutiques choisis dans le groupe consistant en les inhibiteurs d'absorption du cholestérol, les inhibiteurs de protéine de transfert d'ester de cholestérol, les composés pharmaceutiques augmentant les HDL, les statines, leurs dérivés phosphates, et leurs mélanges sont aussi destinés à être administrés.

6. Dérivés phosphates destinés à être utilisés dans un procédé pour abaisser des taux accrus d'un lipide dans le sang circulant selon l'une quelconque des revendications 1 ou 3 à 5 ou utilisation selon l'une quelconque des revendications 2 à 5, où l'abaissement de taux accrus d'un lipide dans le sang circulant atténue les symptômes, traite ou prévient une maladie choisie dans le groupe consistant en la maladie cardiovasculaire, l'athérosclérose, le diabète sucré, la maladie rénale chronique, les hyperlipidémies primaires et secondaires et la dyslipidémie, les rétinopathies, l'hypertrophie du foie et de la rate, les xanthomes et leurs combinaisons.

7. Dérivés phosphates destinés à être utilisés dans un procédé pour abaisser des taux accrus d'un lipide dans le sang circulant selon la revendication 6 ou utilisation selon la revendication 6, où la maladie est choisie dans le groupe consistant en la maladie rénale chronique, les hyperlipidémies primaires et secondaires et la dyslipidémie, les rétinopathies, l'hypertrophie du foie et de la rate, les xanthomes et leurs combinaisons.

# Figure 1

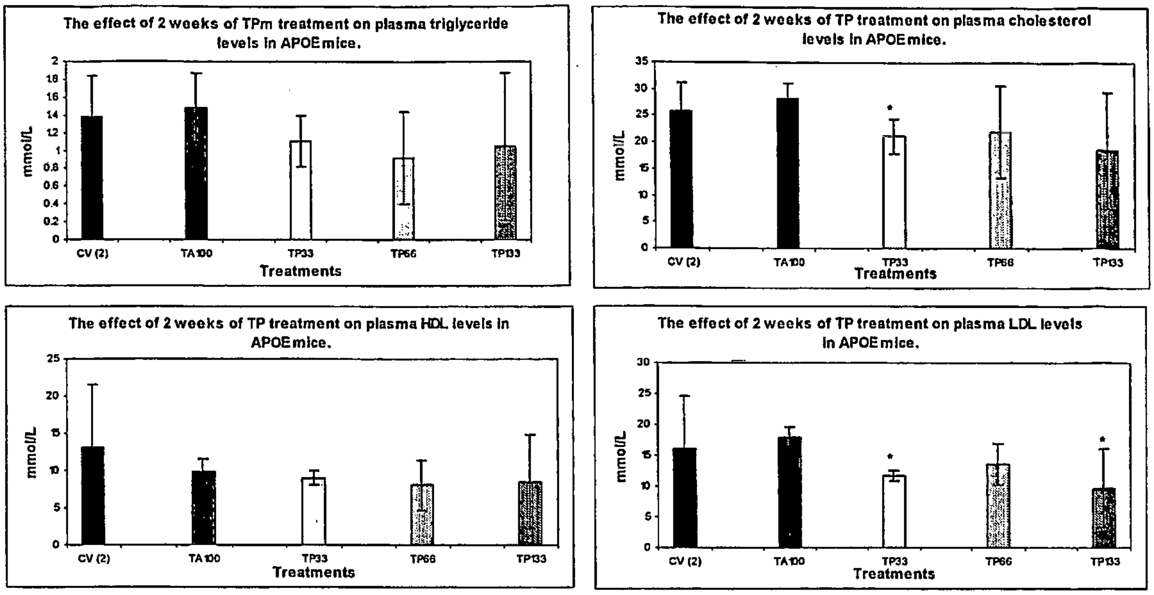


# Figure 2

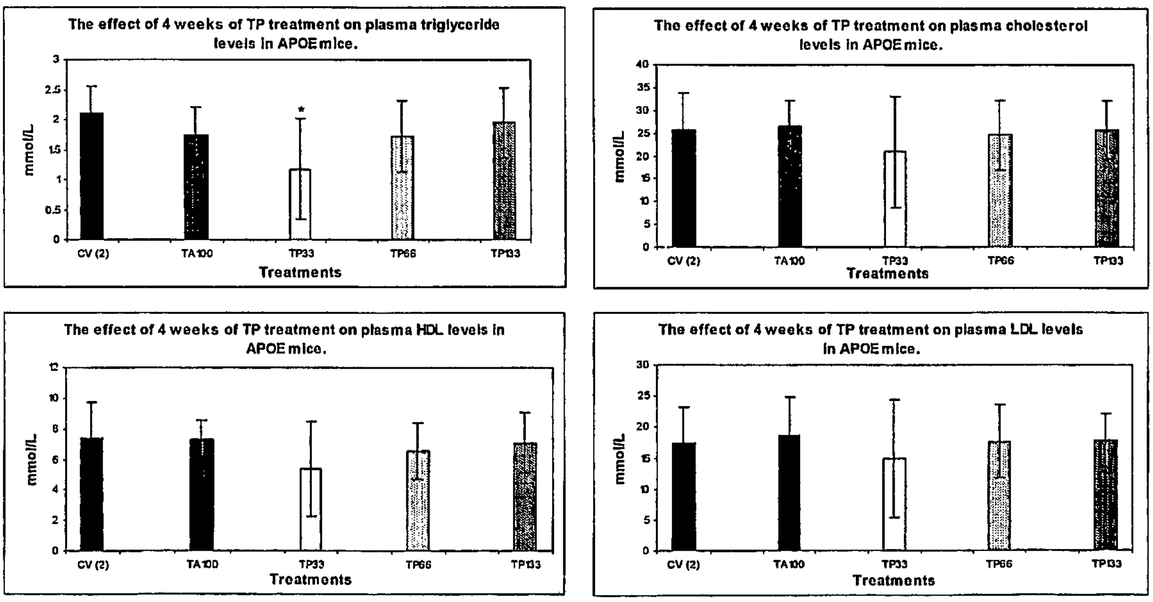

Figure 3

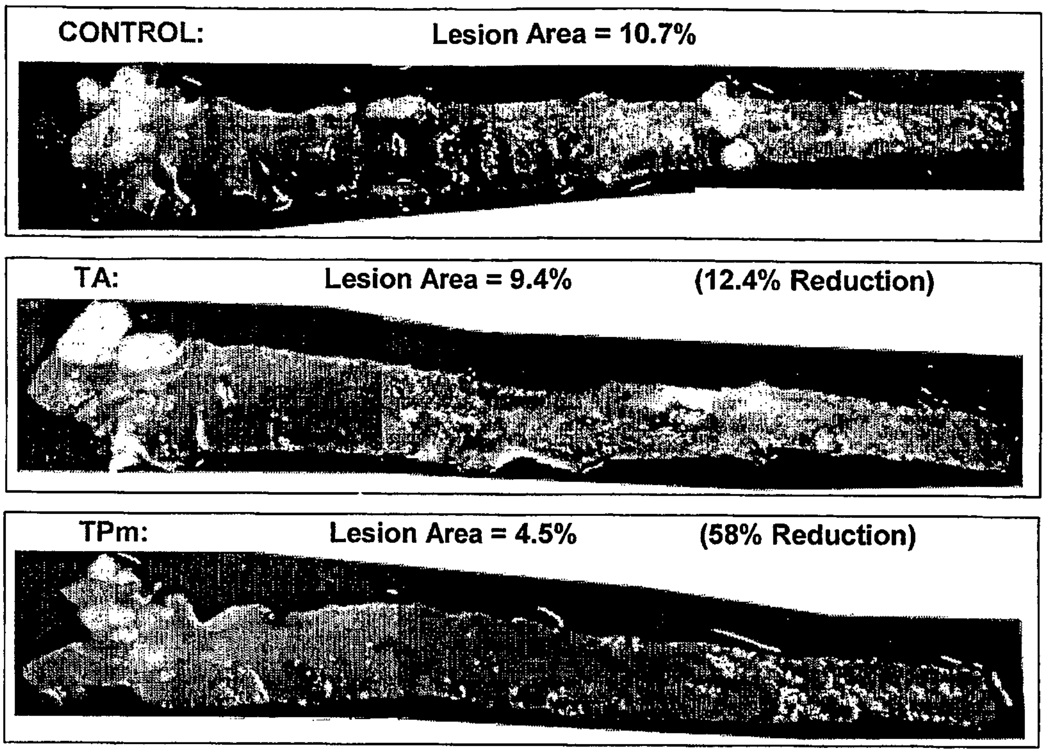
CONTROL:
Lesion Area = 10.7%
TA:
Lesion Area = 9.4%
(12.4% Reduction)
TPm:
Lesion Area = 4.5%
(58% Reduction)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 0617963 A
- WO 03026673 A
- WO 2004064831 A
- AU 0101475 W
- AU 0101476 W
- AU 0101206 W
- AU 0201003 W